# EUROPEAN PATENT APPLICATION

(11) **EP 4 212 154 A1**
(43) Date of publication of application: **19.07.2023**
(21) Application number: 21865518.1
(22) Date of filing: 02.04.2021
(51) Int. Cl.: A61K 31/277, A61K 31/4406, A61P 13/00

(54) **APPLICATION OF ESTER GROUP-CONTAINING AROMATIC PROPIONAMIDE COMPOUNDS IN PREPARATION OF DRUGS FOR TREATING URINARY INCONTINENCE**

(30) Priority: 14.09.2020 CN 202010959518
(71) Applicant: Changchun Genescience Pharmaceutical Co., Ltd., Changchun, Jilin 130012 (CN)
(72) Inventor: ZHU, Xinfa, Zhejiang 315102 (CN); ZHU, Ran, Zhejiang 315102 (CN)
(74) Representative: Gille Hrabal Partnerschaftsgesellschaft mbB Patentanwälte
(86) International application number: PCT/CN2021/085270
(87) International publication number: WO 2022/052455

(57) **Abstract**

Disclosed is an application of ester group-containing aromatic propionamide compounds in preparation of drugs for treating urinary incontinence.

## Description

### TECHNICAL FIELD

The present disclosure belongs to the technical field of medicines, and particularly relates to use of an ester group-containing aromatic propionamide compound for the manufacturing of a medicament for the treatment of urinary incontinence.

### BACKGROUND

Stress urinary incontinence refers to the involuntary leakage of urine when the intravesical pressure exceeds the maximum urethral pressure in the absence of detrusor contraction when the abdominal pressure is increased. Postpartum stress urinary incontinence refers to the stress urinary incontinence symptom occurring only after pregnancy and childbirth, and it is the most common type of female urinary incontinence and greatly affects the health and quality of life of puerpera. There is no effective therapeutic regimen for urinary incontinence so far, and how to ameliorate the urinary incontinence symptoms of patients is of great clinical significance.

### SUMMARY

The object of the present disclosure is to provide use of an ester group-containing aromatic propionamide compound for the manufacturing of a medicament for the treatment of urinary incontinence.

To achieve the object above, the following technical solution is adopted in the present disclosure:
The use of an ester group-containing aromatic propionamide compound for the manufacturing of a medicament for the treatment of urinary incontinence.

Preferably, the urinary incontinence is stress urinary incontinence.

Preferably, the ester group-containing aromatic propionamide compound is prepared in the form of drops.

Preferably, an effective dose range of the ester group-containing aromatic propionamide compound is 0.7-15 mg/kg.

Preferably, the ester group-containing aromatic propionamide compound is C₂₅H₁₇F₃N₄O₄.

The present disclosure has the advantage that the ester group-containing aromatic propionamide compound C₂₅H₁₇F₃N₄O₄ provided herein can markedly ameliorate stress urinary incontinence.

### BRIEF DESCRIPTION OF THE DRAWINGS

FIG. 1 is a schematic diagram showing the comparison of the LPP detection results of the beagle dogs in each group.
FIG. 2 is a schematic diagram showing the comparison of the ALPP detection results of the beagle dogs in each group.
FIG. 3 is a schematic diagram showing the comparison of the MNCV detection results of the beagle dogs in each group.
FIG. 4 is a schematic diagram showing the comparison of the SNCV detection results of the beagle dogs in each group.
FIG. 5 is a schematic diagram showing the comparison of the detection results of the number of urination times of each group.
FIG. 6 is a schematic diagram showing the comparison of the detection results of duration of external urethral sphincter electromyography of each group.
FIG. 7 is a schematic diagram showing the comparison of the detection results of amplitude of external urethral sphincter electromyography of each group.
FIG. 8 is a schematic diagram showing the comparison of the weight results of external urethral sphincter of each group.
FIG. 9 is a schematic diagram showing the comparison of the weight results of ani sphincter of each group.
FIG. 10 is a schematic diagram showing the results of bladder leak point pressure of rat after administration for 21 days.
FIG. 11 is a schematic diagram showing the results of the weight of rat levator ani after administration for 21 days.

### DETAILED DESCRIPTION

The following experiments were conducted to verify the effect of the ester group-containing aromatic propionamide compound of the present disclosure on the treatment of urinary incontinence. The EG compounds mentioned herein are all ester group-containing aromatic propionamide compounds. EG-017 has the chemical formula of C₂₅H₁₇F₃N₄O₄, and reference can be made to the content of patent document with application No. 2014103602169 for its specific preparation method and structural formula.

### Name and objective of experiment

Name of experiment
Effect of EG compounds on ameliorating beagle dogs stress urinary incontinence constructed by vaginal dilation
Objective of experiment
To observe the effect of test substance EG-017 on ameliorating beagle dogs stress urinary incontinence constructed by vaginal dilation

### Name, stability and other characteristics of test substance

Name of test substance: EG-017
Storage conditions of test substance: sealed and stored at room temperature

### Species, number, sex, weight range, source, animal certificate No. and issuing institute, and feeding conditions of experimental animals

Experimental animals
36 female beagle dogs, general grade, weighed 7-17 kg. Animal certificate No.: SCXK (Lu) 20160006, SCXK (Jing) 20160004
Feeding conditions
Feeding room: 12-hour light/dark cycle
Temperature: constant temperature of 20±4 °C
Body weight: recorded once a week

### Grouping of experimental animals

Normal group: 6 dogs; experimental groups: 30 dogs
Animals in the experimental groups were randomly divided into 5 groups according to leak point pressure before modeling, namely model vehicle control group (Model), EG017(L) for 0.7 mg/kg low-dose group, EG017(M) for 2 mg/kg medium-dose group and EG017(H) for 6 mg/kg high-dose group (6 animals in each group).

### Establishment of animal models

Animals in the experimental groups were subjected to modeling twice with an interval of four days. The major steps are as follows: the animals were each anesthetized with Zoletil (5 mg/kg, IM) and Lumianning (2 mg/kg, IM) and fixed in a supine position, the bladder was drained by using a sterile catheter, the part with the balloon at the head end of the 24F catheter was placed in the canine vagina (the depth was about 10cm), a certain amount (about 40 mL, the input amount varied due to individual difference of the animals, as long as the balloon did not slide out) of sterile normal saline was injected into the balloon by using an injector to prepare a vaginal dilation balloon, and a heavy object (350 mL of normal saline) was suspended at the drooping tail end of the catheter; the normal group was left untreated, and for the model dogs, the normal saline in the balloon was drained after a specified time period (4 hours), the catheter was slowly removed, and local disinfection was performed.

### Detection of leak point pressure

A multi-channel physiological signal acquisition and processing system (Chengdu Instrument Factory) was used for detection before modeling, after modeling, and 2 weeks and 4 weeks after drug administration.

### Urodynamic detection

The main procedures were as follows:
Treatment before detection: the beagle dogs were anesthetized with Zoletil (5 mg/kg, IM) and Lumianning (2 mg/kg, IM) and then placed in a supine position, the forelimbs were fixed at the two corners of the operating table, the urethral orifice was disinfected with iodophor, and an epidural catheter coated with vaseline was inserted into the bladder through the urethra and used as the bladder piezometric tube and infusion tube. The piezometric tube was connected with a sensor of a urodynamic detector through a three-way valve. The animal bladder was firstly drained, the infusion tube was connected with a micro-injection pump through the three-way valve, and the water pumping speed was 30-50 mL/min.

Detection of pressure in the bladder in filling period: after the urodynamic detector was set to zero *in vivo,* the micro-injection pump was started to inject room-temperature normal saline into the bladder; when the first drop of liquid appeared at the external orifice of the urethra, the injection was stopped and the bolus amount of the injector was recorded; the injection volume recorded at this moment was the maximum volume of the bladder, and the recorded intravesical pressure was the leak point pressure (LPP).

### Detection of abdominal leak point

Following urodynamic detection, the animal's bladder was first drained and room-temperature normal saline was injected using a micro pump in an amount of one-half of the maximum bladder capacity. The pressure applied to the head side of the bladder was increased slowly, and the urethral orifice was observed until the first drop of liquid appeared. The pressure value showed by the signal acquisition system at this moment was the abdominal leak point pressure (ALPP).

### Detection of coccygeal nerve conduction velocity

The coccygeal nerve conduction velocity was detected using a multi-channel physiological signal acquisition and processing system (Chengdu Instrument Factory) before modeling, after modeling, and 2 weeks and 4 weeks after drug administration. The main procedures were as follows:
After the animals were anesthetized, the animals were fixed in a lateral decubitus position, and the skin at the tail part was cleaned and degreased with 75% ethanol. An acupuncture needle was used as a stimulation electrode and inserted into the near end of the tail part, a recording electrode was inserted into the muscle at the far end of the tail part and was close to the tail vein on the lateral side, and a reference electrode was placed at the far end of the tail part and was about 2 cm away from the recording electrode. The waveform was collected when the stable composite action potential was recorded, and the distance between the stimulation electrode and the recording electrode was measured. The action potential latency was detected, and the motor nerve conduction velocity (MNCV) was calculated by distance/duration of latency; the sensory nerve conduction velocity (SNCV) can be obtained by the same method when the positions of the stimulating and recording electrodes were exchanged.

### Monitoring of number of urination times

The number of urination times was monitored by laying a urine pad at the bottom of the feeding cage. The number of urination times of the animals within 24 hours was observed and counted.

Monitoring frequency: before modeling, after modeling, and after the administration of 2 weeks and 4 weeks.

### External urethral sphincter electromyography monitoring and tissue collection: performed at experimental endpoint

The method was specifically as follows: recording of external urethral sphincter electromyography (EUS EMG): the urethra was exposed, a medical double needle electrode was placed at the urethral sphincter, and the electrode was connected to a biological signal acquisition system (a multi-channel physiological signal acquisition system, Chengdu Instrument Factory). Maximum voltage stimulation was applied, the electromyogram of the external urethral sphincter was simultaneously acquired, and statistical analysis of the maximum amplitude and duration of the BUS EMG was performed. The animals were dissected, and the ani sphincter (AS) and external urethral sphincter (about 1 cm in length) were collected and weighed.

### Preparation of formulation and administration

Preparation of EG017: the administration doses for animals were 0.7 mg/kg, 2 mg/kg and 6 mg/kg, and the administration volume was 5 mL/kg. EG017 was weighed out according to the body weight of the animal, and the vehicle, 0.5% CMC-Na, was added to prepare suspensions with final concentrations of 0.14 mg/mL, 0.4 mg/mL and 1.2 mg/mL. EG017 was freshly prepared and used on the day of the experiment. During the administration period of experimental animals, the suspension was stirred by a magnetic stirrer, so that the suspension was always in a stirring state and the drug was well mixed.

### Instruments used

Precision electronic balance (Ohaus Instrument (Changzhou) Co., Ltd., EX224ZH); electronic scale (GSS Scale (Suzhou) Co., Ltd., TSC-21); disposable sterile catheter (Changshu Shenling Medical Apparatus Co., Ltd., F24).

### Data processing

All data were expressed as Mean±SEM. For the experimental data, the GraphPad Prism 5t test was used to analyze and compare the data of the groups. For the statistical analysis results, P < 0.05 indicates that the difference was statistically significant, P < 0.01 indicates significant difference, and P < 0.001 indicates extremely significant difference; * represents each group vs Model group, and # represents D15 and D29 of each group vs D1 of each group.

### Experimental results

### LPP detection results of beagle dogs in each group

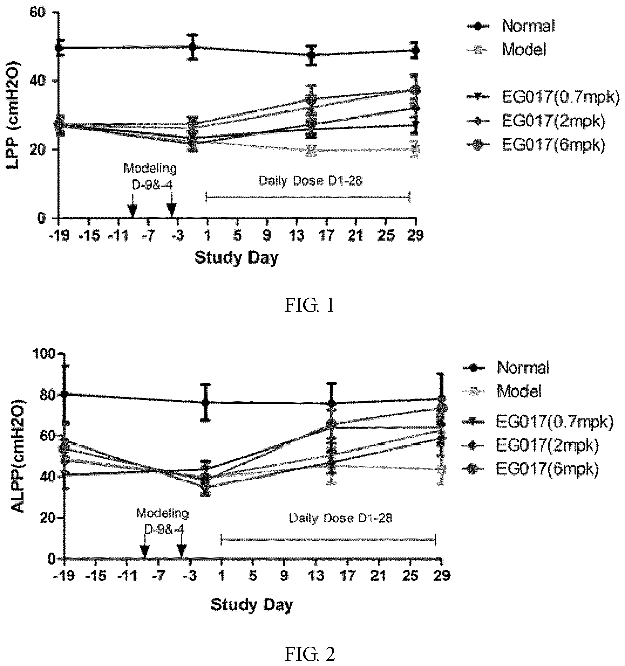

As shown in FIG. 1, according to the results of D14 administration (detected on D15, the same below), the LPP of the EG017 (0.7 mpk) group and that of the EG017 (2 mpk) group were markedly increased compared with that of the Model group, and the difference was statistically significant (P < 0.05); the LPP of the EG017 (6 mpk) group was significantly increased compared with that of the Model group (P < 0.01); according to the results after D28 administration (detected on D29, the same below), the EG017 (2 mpk) group and the EG017 (6 mpk) groups showed significant increase compared with the Model group (P < 0.01), and each group showed significant increase compared with the result on D-1 (P < 0.05).

### ALPP detection results of beagle dogs in each group

As shown in FIG. 2, according to the results of D28 administration, the ALPP of the EG017 (6 mpk) group was significantly increased compared with that of the Model group (P < 0.01), and both the EG017 (0.7 mpk) group and the EG017 (2 mpk) group showed a trend of increase; both the EG017 (0.7 mpk) group and the EG017 (2 mpk) group showed great increase on D28 (P < 0.05) compared with the results on D-1, and the EG017 (6 mpk) group showed significant increase on both D14 (significant difference, P < 0.01) and D28 (extremely significant difference, P < 0.001) compared with the results on D-1.

### MNCV detection results of beagle dogs in each group

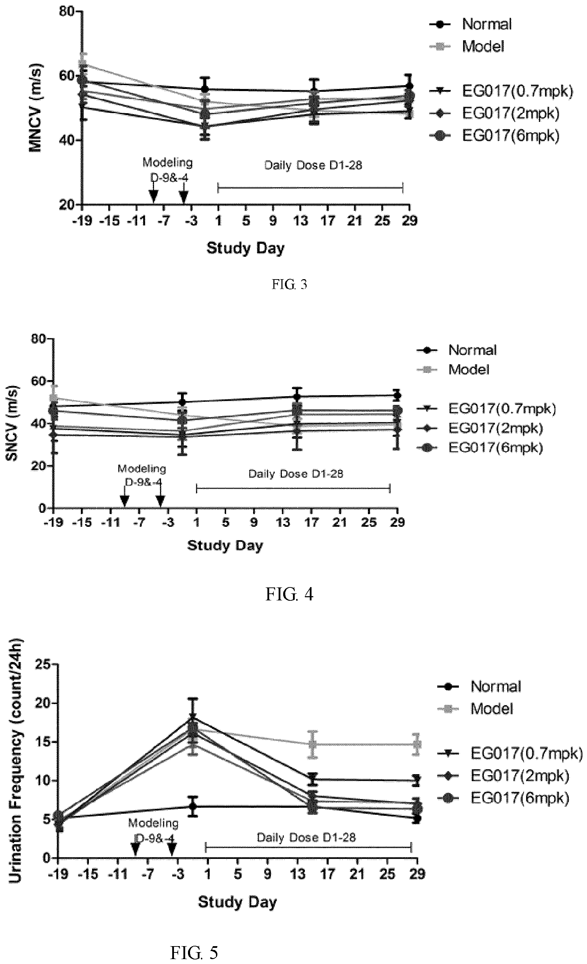

As shown in FIG. 3, according to the results of D28 administration, the EG017 (6 mpk) group showed significant increase in MNCV compared with the Model group, and the difference was statistically significant (P < 0.05).

### SNCV detection results of beagle dogs in each group

As shown in FIG. 4, no statistic difference was found with respect to SNCV between each administration group and the Model group.

### Detection results of number of urination times of each group

As shown in FIG. 5, according to the results of D14 administration, the EG017 (0.7 mpk) group showed great reduction in the number of urination times compared with the Model group, and the difference was statistically significant (P < 0.05), and the EG017 (2 mpk) and the EG017 (6 mpk) group showed significant reduction in the number of urination times compared with the Model group (P < 0.01); according to the results of D28 administration, the EG017 (0.7 mpk) group showed significant reduction in the number of urination times (P < 0.01); both the EG017 (2 mpk) group and the EG017 (6 mpk) group showed extremely significant reduction (P < 0.001); the EG017 (0.7 mpk) group showed great reduction on D14 and D28 compared with the results on D-1 (P < 0.01), and the EG017 (2 mpk) group and the EG017 (6 mpk) group showed extremely significant reduction on D14 and D28 compared with the results on D-1 (P < 0.001).

### Detection results of duration of external urethral sphincter electromyography of each group

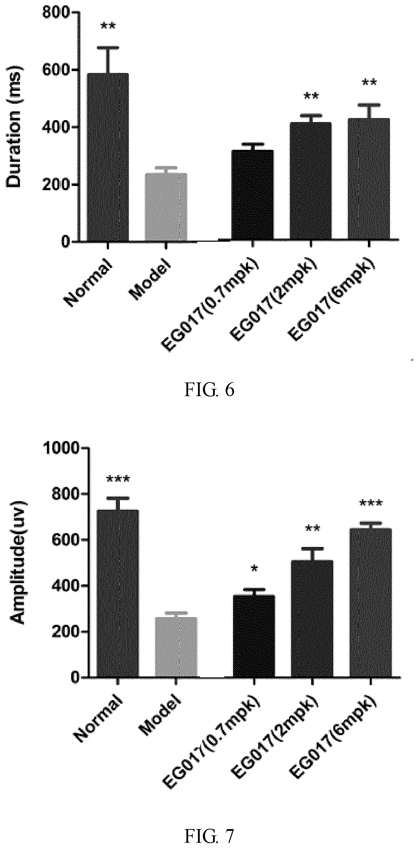

As shown in FIG. 6, according to the results of D28 administration, the EG017 (2 mpk) group and the EG017 (6 mpk) group showed significant increase in the duration of external urethral sphincter electromyography compared with the Model group (P < 0.01).

### Detection results of amplitude of external urethral sphincter electromyography of each group

As shown in FIG. 7, according to the results of D28 administration, the EG017 (0.7 mpk) group showed great increase in the amplitude of external urethral sphincter electromyography compared with the Model group, and the difference was statistically significant (P < 0.05); the EG017 (2 mpk) group showed significant increase (P < 0.01), and the EG017 (6 mpk) group showed extremely significant increase (P < 0.001).

### Weight results of external urethral sphincter of each group

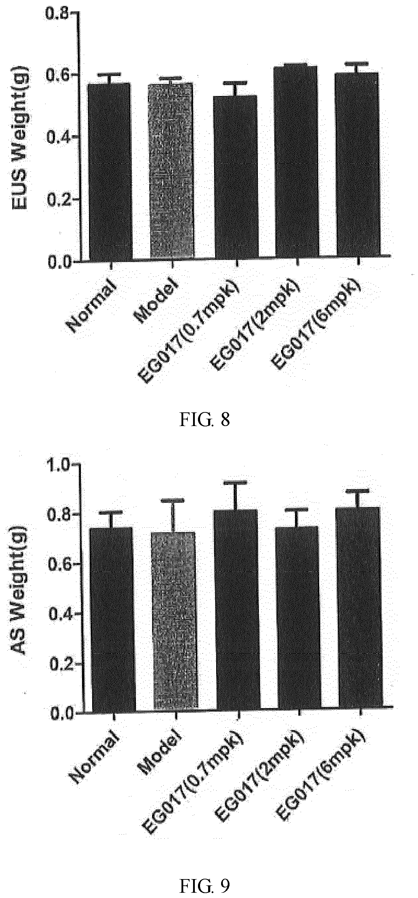

As shown in FIG. 8, no statistic difference was found between each group and the Model group.

### Weight results of ani sphincter of each group

As shown in FIG. 9, no statistic difference was found between each group and the Model group.

Conclusion: under the conditions of the laboratory, EG017 can markedly ameliorate the stress urinary incontinence of beagle dogs.

To further verify the effect of EG017 on the treatment of urinary incontinence, the following experiment was conducted at different doses for urinary incontinence in rats.

### Experimental instruments and materials

Multi-channel signal acquisition and processing system: model: RM-6240CD; manufacturer: Chengdu Instrument Factory.
Scaler-type pressure transducer: model: YPJ01H; manufacturer: Chengdu Instrument Factory.
Weight balance: model: MP5002; manufacturer: Shanghai Sunny Hengping Scientific Instrument Co., Ltd.
Electronic balance: model: SQP-type SECURA225D-ICN; manufacturer: Sartorius Scientific Instruments (Beijing) Co., Ltd. Disposable epidural anesthesia catheter: model: RD (flexible type); manufacturer: Linyi Xinghua Medical Equipment Co., Ltd.
Disposable latex urinary catheter: model: 8#; manufacturer: Well Lead Medical Co., Ltd., Guangzhou

### Major test drug and reagents

### Test compound

Name: EG017; storage condition: stored at room temperature; supplier/manufacturer: 2Y-Chem, Ltd.

### Solvent

Name: sodium carboxymethylcellulose; storage condition: room temperature; preventive measures: see MSDS; supplier/manufacturer: Sigma

### Preparation of solvent and compound

The solvent for the test compound was 0.5% sodium carboxymethylcellulose solution.

0.5% sodium carboxymethylcellulose solution: 5009 mg of sodium carboxymethylcellulose was weighed out and added to deionized water, and the mixture was stirred in a vortex mode until the mixture was fully mixed. The volume was made up to 1001.8 mL.

1.5 mg/kg EG017: 4.51 mg of EG017 was weighed out and dissolved in 0.5% sodium carboxymethylcellulose solution, and the volume was made up to 14.78 mL; the EG017 was used for oral administering after being fully dissolved. 5 mg/kg EG017: 13.02 mg of EG017 was weighed out and dissolved in 0.5% sodium carboxymethylcellulose solution, and the volume was made up to 12.8 mL; the EG017 was used for oral administering after being fully dissolved.

15 mg/kg EG017: 37.65 mg of EG017 was weighed out and dissolved in 0.5% sodium carboxymethylcellulose solution, and the volume was made up to 12.34 mL; the EG017 was used for oral administering after being fully dissolved.

The method for preparing drugs for the remaining 21 days is the same as above.

### Experimental animals

Species: rat; strain: SD; sex: female; month age at start of experiment: 6-8 months old; food supply: free choice feeding; water supply: free choice feeding; cage separation: two for each cage; marking: animal tail marking

### Procedures of animal experiment

### Operation for modeling

Parous female rats were each weighed, anesthetized, and then fixed in a supine position on an operation plate. A No. 8 catheter was inserted into the vagina of the rat at a depth of 2-3 cm, 5mL of sterile normal saline was injected into an balloon, labia majora on two sides were sutured by using a 5-0 silk thread in an "8" shape to prevent the water bag from sliding out, and the tension in a parallel radial direction was applied to the vagina (pulled by a 0.3 kg sand bag) for 4 hours; 4 hours later, the catheter was taken out, and the rat was allowed to be in the natural body position and fed as usual.

Normal nonparous female rats were used as controls and were not treated with operation.

### Determination of base value

### Determination of maximum bladder volume and leak point pressure

The next day after the operation, the rat was anesthetized with gaseous isoflurane and fixed in a supine position. The urethral orifice was disinfected, the self-made epidural catheter was coated with vaseline for lubrication and then inserted into the bladder via the urethra, and the urethral orifice was fixed with an artery clamp to prevent the epidural catheter from sliding out. The epidural catheter was connected to a pressure transducer of a urodynamic detector through a three-way valve to detect the intravesical pressure. Then the bladder was drained through the catheter, the normal saline at 37°C was injected slowly through an injector, the maximum bladder volume was obtained through the reading of the injector when the first drop of urine appeared at the external orifice of the urethra, and the measurement was performed 3 times to obtain an average value; the intravesical pressure recorded at this moment was the leak point pressure (LPP), and the measurement was also performed 3 times to obtain an average value.

### Sneeze test

After the determination of the bladder volume was finished, the bladder was drained and a sneeze test was performed. The rat was in a supine position, the forelimbs were fixed, an epidural catheter was inserted into the bladder via the urethra, and the normal saline was injected until the injection volume reached two thirds of the maximum bladder volume. A purpose-made brush pen was inserted into the nostril of the rat until the sneeze reflection occurred. The urethral orifice of the rat was closely observed. The sneeze test was positive if liquid flowed out of the urethral orifice when sneezing, and the sneeze test was negative if no liquid flowed out of the urethral orifice when sneezing.

### Experimental grouping and administration:

### Grouping

50 rats positive in sneeze test were selected and randomly divided into 5 groups according to the leak point pressure of model rats.

10 normal nonparous female rats constituted a control group. The situation of each group was shown in the table below:

| Group | Administration | Dose (mg/kg) | Administration volume (mL/kg) | Route of administration | Number of animals |
|---|---|---|---|---|---|
| 1 | Normal control group | - | - | - | 10 |
| 2 | Solvent control group | - | 5 | Oral administration | 10 |
| 3 | EG017 | 1.5 | 5 | Oral administration | 10 |
| 4 | EG017 | 5 | 5 | Oral administration | 10 |
| 5 | EG017 | 15 | 5 | Oral administration | 10 |

### Administration:

The animals above were subjected to administration once daily for 21 consecutive days, and the endpoint test was performed on day 22.

### Post-administration endpoint test

### Determination of maximum bladder volume and leak point pressure

The rat was anesthetized with gaseous isoflurane and fixed in a supine position. The urethral orifice was disinfected, the self-made epidural catheter was coated with vaseline for lubrication and then inserted into the bladder via the urethra, and the urethral orifice was fixed with an artery clamp to prevent the epidural catheter from sliding out. The epidural catheter was connected to a pressure transducer of a urodynamic detector through a three-way valve to detect the intravesical pressure. Then the bladder was drained through the catheter, the normal saline at 37°C was injected slowly through an injector, the maximum bladder volume was obtained through the reading of the injector when the first drop of urine appeared at the external orifice of the urethra, and the measurement was performed 3 times to obtain an average value; the intravesical pressure recorded at this time point was the leak point pressure (LPP), and the measurement was also performed 3 times to obtain an average value.

### Sneeze test

After the determination of the bladder volume was finished, the bladder was drained and a sneeze test was performed. The rat was in a supine position, the forelimbs were fixed, an epidural catheter was inserted into the bladder via the urethra, and the normal saline was injected until the injection volume reached two thirds of the maximum bladder volume. A purpose-made brush pen was inserted into the nostril of the rat until the sneeze reflection occurred. The urethral orifice of the rat was closely observed. The sneeze test was positive if liquid flowed out of the urethral orifice when sneezing, and the sneeze test was negative if no liquid flowed out of the urethral orifice when sneezing.

### Weighing of rat levator ani muscle

After the sneeze test was completed, the animals were each euthanized, and the rat levator ani on one side (left side) was separated out and weighed.

### Data collection and analysis

All data were expressed as Mean±SEM. For the statistics of experimental data, the one-way ANOVA and the Dunnett's multiple comparison test were used to analyze and compare the data of the groups. For the statistical analysis results, P < 0.05 indicates significant difference and is expressed with *; P < 0.01 indicates very significant difference and is expressed with **; P < 0.001 indicates extremely significant difference and is expressed with ***.

### Results

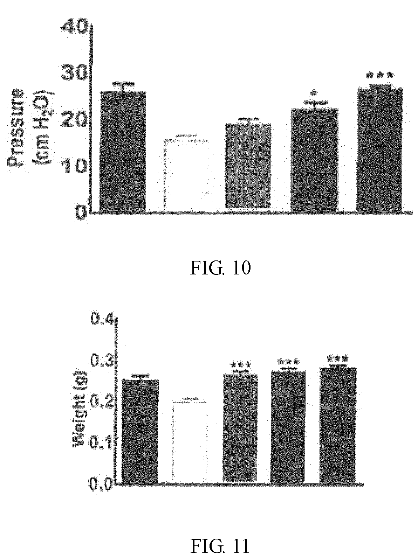

After 21 days of oral administration, the compound EG017 could markedly ameliorate the urinary incontinence symptoms of rats and showed dose dependence. The results showed that: for the EG017 at 1.5 mg/kg dose, the positive rate of the rat sneeze test was reduced from 100% to 40%; for the EG017 at 5 mg/kg dose, the positive rate of the rat sneeze test was reduced from 100% to 40%; for the EG017 at 15 mg/kg dose, the positive rate of the rat sneeze test was reduced from 100% to 20%; the data in FIGs. 10 and 11 show that EG017 was able to dose-dependently increase the leak point pressure of rat bladder (*p < 0.05, ***p < 0.001) and increase the weight of rat levator ani muscle (*p < 0.05, **p < 0.01, ***p < 0.001), indicating that EG017 has a very good therapeutic effect on urinary incontinence. In the drawings related to this example, the sequence of schematic columns is based on the sequence of groups in the table.

## Claims

1. Use of an ester group-containing aromatic propionamide compound for the manufacturing of a medicament for the treatment of urinary incontinence.

2. The use of claim 1, wherein the urinary incontinence is stress urinary incontinence.

3. The use of claim 3, wherein the ester group-containing aromatic propionamide compound is prepared in the form of drops.

4. The use of claim 3, wherein an effective dose range of the ester group-containing aromatic propionamide compound is 0.7-15 mg/kg.

5. The use of claim 4, wherein the ester group-containing aromatic propionamide compound is C₂₅H₁₇F₃N₄O₄.
